# FASCICULE DE BREVET EUROPEEN

(11) **EP 1 231 893 B1**
(45) Date de publication et mention de la délivrance du brevet: **18.08.2004**
(21) Numéro de dépôt: 00985316.9
(22) Date de dépôt: 24.11.2000
(51) Int. Cl.: A61K 7/48

(54) **EXTRAIT D'AJUGA TURKESTANICA ET SES APPLICATIONS COSMETIQUES**
AJUGA TURKESTANIKA EXTRAKT UND SEINE KOSMETISCHE VERWENDUNG
AJUGA TURKESTANICA EXTRACT AND ITS COSMETIC USES

(30) Priorité: 26.11.1999 FR 9914893
(43) Date de publication de la demande: 21.08.2002
(73) Titulaire: LVMH RECHERCHE, 75008 Paris (FR)
(72) Inventeur: Dumas, Marc Apt. 61 61, Rce "Le Windsor", 45100 Orleans (FR); Bonte, Frédéric, 45100 Orleans (FR); Gondran, Catherine, 45730 St Benoit-sur-Loire (FR)
(74) Mandataire: Giraud, Françoise
(86) Numéro de dépôt international: PCT/FR2000/003274
(87) Numéro de publication internationale: WO 2001/037799

(56) Documents cités:
- EP-A- 0 440 494
- WO-A-94/04132
- US-A- 3 527 777
- A.U.MAMATKHANOV ET AL.: "iSOLATION OF TURKESTERONE FROM THE EPIGEAL PART OF AJUGA TURKESTANICA AND IRS ANABOLIC ACTIVITY" CHEM. NAT. COMPD., vol. 34, no. 2, 1998, pages 150-154, XP000925904
- DATABASE CHEMABS [en ligne] CHEMICAL ABSTRACTS SERVICE, COLUMBUS, OHIO, US; vol.123 abstract no.296372, KOTENKO, L.D.ET AL.: "MONITORING OF TOTAL IRIDOID PRODUCTION BY AJUGA TURKESTANICA" XP002148031 & KHIM. PRIR. SOEDIN., vol. 6, 1994, pages 824-5,

## Description

La présente invention concerne un nouvel extrait de la plante Ajuga turkestanica ainsi que ses applications dans le domaine cosmétique.

On a décrit dans le brevet français FR 2 696 075 l'utilisation d'ecdystéroïdes pour renforcer la barrière hydrique de la peau du fait de leur action sur la différenciation des kératinocytes. Ce brevet cite un grand nombre de sources d'ecdystéroïdes aussi bien dans le domaine animal que végétal. Parmi ces nombreuses sources, on trouve, en particulier, deux plantes de la famille des Ajuga, à savoir Ajuga iva et Ajuga decumbens.

La plante Ajuga turkestanica est une plante de la famille des Labiae, du genre Ajuga, espèce turkestanica qui croit en Asie centrale, en particulier en Ouzbékistan. Son nom ouzbek traditionnel est "sanobar", la charmeuse.

Cette plante était utilisée en médecine traditionnelle sous forme de tisane pour ses propriétés tonifiantes et hépatoprotectrices.

Une étude récente intitulée "Effect of a lipid concentrate from the aboveground portion of Ajuga turkestanica on the metabolic processes and dynamics of healing skin wounds experimentally", Pharmaceutical Chemistry Journal, Vol. 28, No. 11, 1994, 837-840, a relaté des essais pharmacologiques réalisés à partir d'extraits chloroformiques des parties aériennes de cette plante. Parmi les résultats relatés dans cette publication, il est apparu que les extraits présentaient une certaine activité d'hydratation des tissus.

Les inventeurs de la présente invention ont maintenant découvert qu'il était possible, à condition d'utiliser des solvants nettement plus polaires que ceux utilisés dans le cadre de cette étude expérimentale, d'obtenir des extraits très différents présentant non seulement des propriétés remarquablement améliorées d'hydratation de l'épiderme liées d'une part à l'amélioration de la différenciation des kératinocytes conduisant au renforcement de la barrière hydrique de la peau mais également à une hydratation dite "hydratation active" de l'épiderme par ré-absorption d'eau et amélioration des flux hydriques dans l'épiderme.

Les extraits qui constituent en eux-mêmes des produits nouveaux constituent le premier objet de la présente invention qui concerne également, selon d'autres objets, des compositions cosmétiques et des utilisations de ces extraits dans le domaine de la cosmétique.

Par ailleurs, poursuivant leurs recherches, les inventeurs de la présente invention ont mis en évidence que, parmi les extraits de l'invention, certains contenaient des associations d'actifs bien déterminés dans des proportions bien déterminées ci-après désignées par "associations" de l'invention, jamais utilisées à ce jour dans des compositions cosmétiques. Les compositions cosmétiques contenant ces associations ainsi que les utilisations cosmétiques de ces associations constituent donc un autre objet de l'invention.

Les inventeurs de la présente invention ont déduit des propriétés des extraits et associations de l'invention que ces extraits et associations étaient particulièrement actifs pour réguler et/ou améliorer la fonctionnalité des aquaporines AQP3.

Les aquaporines ou canaux hydriques sont des systèmes protéiques transmembranaires transporteurs d'eau et de petites molécules en solution telles que le glycérol et l'urée par exemple. La taille de ces aquaporines est d'environ 30 kDa avec 6 passages trans membranaires en hélice alpha (Jung et al. "Molecular structure of the water channel through aquaporin CHIP" dans J. Biol. Chem. 1994 ; vol. 269, pp. 14648-14654).

La présence d'aquaporines de type 3 ou AQP3 dans l'épiderme humain et, plus précisément dans la membrane plasmique des kératinocytes de la peau humaine a été décrite par R. SOUGRAT et al. dans un article "Functional expression of AQP3 in human epidermis and keratinocyte cell cultures" publié dans Molecular Biology of the Cell ; Nov. 1998 ; vol. 9, page 499a. R. SOUGRAT et al. a également décrit dans cet article le rôle important que jouent ces AQP3 dans le transport de l'eau au sein de l'épiderme humain.

Ainsi donc, l'invention résulte de la découverte de ce que, en sélectionnant des milieux solvants particulièrement polaires, il était possible d'obtenir des extraits de la plante Ajuga turkestanica présentant des propriétés cosmétiques particulièrement intéressantes et de ce que parmi ces extraits certains contiennent des associations d'actifs dont l'utilisation cosmétique conduit aux mêmes propriétés cosmétiques remarquables. Ces extraits et associations permettent, en particulier, d'améliorer la différenciation des kératinocytes, ce qui permet d'améliorer l'hydratation de la peau et d'obtenir une activité anti-vieillissement particulièrement efficace. Par ailleurs, les inventeurs de la présente invention ont pu observer que les extraits et associations de l'invention avaient une efficacité particulière pour réguler les flux hydriques dans l'épiderme, activité qu'ils ont pu relier à une remarquable activité des extraits de l'invention sur la régulation et/ou la fonctionnalité des aquaporines AQP3, permettant ainsi une meilleure hydratation des couches basales de l'épiderme.

Ainsi, selon l'une de ses caractéristiques essentielles, l'invention concerne un extrait de la plante Ajuga turkestanica, caractérisé en ce qu'il contient au moins un ecdystéroïde et au moins un iridoïde et en ce qu'il est susceptible d'être obtenu par extraction d'une partie au moins de ladite plante au moyen d'un solvant ou d'un mélange de solvants constitué de 0 à 60 % en poids d'eau, le reste dudit solvant ou mélange de solvants étant constitué d'au moins un alcool en C₁ à C₄, et/ou de l'acétone et/ou du butylène glycol et/ou du propylène glycol.

Le procédé d'extraction utilisable pour préparer les extraits de l'invention est avantageusement mis en oeuvre dans les conditions telles que ladite extraction est réalisée avec ledit solvant ou mélange de solvants pendant 1 à 4 heures.

Le procédé d'extraction utilisable pour préparer les extraits de l'invention est avantageusement mis en oeuvre dans les conditions telles que ladite extraction est réalisée au reflux dudit solvant ou mélange de solvants pendant 1 à 4 heures.

L'extraction peut être réalisée sur la plante entière ou sur une partie de la plante. Toutefois, on préférera utiliser pour la préparation des extraits de l'invention les racines de la plante.

Les proportions d'eau et d'alcool ou d'eau et d'acétone dans le solvant ou mélange de solvants utilisé pour la mise en oeuvre du procédé d'extraction permettant de préparer les extraits de l'invention peuvent varier dans de larges proportions. Toutefois, on préférera pour les solvants alcooliques ou l'acétone une proportion d'eau comprise entre 0 et 50% en poids

Les proportions d'eau et de butylène glycol ou de propylène glycol dans le solvant ou mélange de solvants utilisé pour la mise en oeuvre du procédé d'extraction permettant de préparer les extraits de l'invention peuvent varier dans de larges proportions. Toutefois, on préférera pour le butylène glycol ou le propylène glycol une proportion proche de 50% d'eau en poids.

Le mélange de solvants utilisé pour la mise en oeuvre du procédé d'extraction permettant de préparer les extraits de l'invention peut être composé d'un mélange quelconque d'alcools en C₁ à C₄, d'eau, de butylène glycol ou de propylène glycol.

A titre d'alcools préférés pour la mise en oeuvre du procédé permettant de préparer les extraits de l'invention, on citera essentiellement le méthanol et l'éthanol.

Pour la préparation des extraits selon l'invention, on recourra avantageusement à des mélanges eau-éthanol, eau-méthanol, eau-acétone, eau-propylène glycol ou eau-butylène glycol.

Dans le cas des mélanges eau-éthanol, on choisira avantageusement un mélange contenant 70 à 90 % d'éthanol et 30 à 10 % d'eau, de préférence un mélange contenant 80 % d'éthanol pour 20 % d'eau.

Dans le cas des mélanges eau-méthanol, on choisira avantageusement un mélange contenant 70 à 90 % de méthanol et 30 à 10 % d'eau, de préférence un mélange contenant 80 % de méthanol pour 20 % d'eau.

Dans le cas des mélanges eau-acétone, on choisira avantageusement un mélange contenant 70 à 90 % d'acétone et 30 à 10 % d'eau, de préférence un mélange contenant 80 % d'acétone pour 20 % d'eau.

Dans le cas des mélanges eau-propylène glycol, on choisira avantageusement un mélange contenant 40 à 60 % de propylène glycol et 60 à 40 % d'eau, de préférence 50 % de propylène glycol pour 50 % d'eau.

Dans le cas des mélanges eau-butylène glycol, on choisira avantageusement un mélange contenant 40 à 60 % de butylène glycol et 60 à 40 % d'eau, de préférence 50 % de butylène glycol pour 50 % d'eau.

Il est apparu que les extraits de l'invention contiennent avantageusement des associations contenant une partie en poids d'au moins un ecdystéroïde et 2 à 4 parties en poids, de préférence 3 parties en poids, d'au moins un iridoïde.

Ces associations sont ci-après dénommées par simplification "associations" selon l'invention.

Selon une variante avantageuse, les ecdystéroïdes contenus dans ces associations sont choisis de préférence dans le groupe constitué par l'ecdystérone, le 2,3-monoacétonide d'ecdystérone, la turkestérone, la cyastérone, la 22-acétyl-cyastérone et l'alpha-ecdysone.

Selon une autre variante avantageuse, les iridoïdes. contenus dans ces associations sont choisis dans le groupe constitué par l'harpagide et son dérivé 8-O-acétylé.

Selon une variante avantageuse, les différents ecdystéroïdes contenus dans ces associations sont composés, exprimés en poids de 30 à 35 % d'ecdystérone, 20 à 25 % de turkestérone, 20 à 24 % de 22-acétyl-cyastérone, 11 à 15 % de 2,3-monoacétonide d'ecdystérone, 6 à 7 % d'alpha-ecdysone, 2 à 4 % de cyastérone.

Selon une autre variante avantageuse, les iridoïdes sont composés, exprimés en poids, de 60 à 70 % de 8-O-acétyl-harpagide et de 30 à 40 % d'harpagide.

Comme exposé précédemment, les extraits de l'invention présentent une activité particulière de différenciation des kératinocytes, ce qui leur permet de contribuer au renforcement du rôle de barrière hydrique de l'épiderme, en améliorant ainsi son hydratation et en jouant un rôle anti-vieillissement, améliorant l'aspect et la qualité de la peau, permettant ainsi d'obtenir une belle peau, une peau douce.

Les essais réalisés par les inventeurs de la présente invention ont montré que les remarquables propriétés des extraits de l'invention étaient également obtenues avec les associations d'ecdystéroïde et d'iridoïde particulières telles que définies ci-dessus.

Ainsi donc, selon un deuxième aspect, la présente invention concerne l'utilisation d'un extrait de la plante Ajuga turkestanica ou d'une association d'au moins un ecdysteroïde et d'au moins un iridoïde telle que définie précédemment comme agent cosmétique améliorant la différenciation des kératinocytes. Cette activité sur la différenciation des kératinocytes conduit à améliorer la fonction de barrière hydrique de l'épiderme et, par conséquent, à un effet hydratant, à un effet anti-vieillissement, et permet également d'améliorer l'aspect et la qualité de la peau pour fournir, en particulier, une belle peau, une peau plus douce.

Comme exposé précédemment, les essais réalisés par les inventeurs ont permis de mettre en évidence l'activité des extraits de l'invention et des associations définies précédemment sur le transport d'eau dans l'épiderme, activité reliée au moins en partie à une action, également mise en évidence par les inventeurs de l'invention, sur la régulation et/ou la fonctionnalité des aquaporines AQP3.

En effet, il est bien connu que, lors du vieillissement de la peau, elle devient plus fine, plus sèche donnant cet aspect fin avec écailles rencontré en particulier chez les personnes âgées. Dans la peau il est bien connu qu'il existe un gradient d'eau de la couche basale de l'épiderme aux couches les plus superficielles du stratum corneum. Or, les inventeurs de la présente invention ont, après avoir étudié le transport d'eau sur des cultures émergées de kératinocytes humains normaux traitées et témoins, mis en évidence par des mesures de perméabilité que les extraits de l'invention permettaient d'augmenter les flux d'eau dans l'épiderme et notamment les flux de ré-absorption de l'eau vers la couche basale de l'épiderme conduisant à une meilleure hydratation de l'épiderme et des couches basales de l'épiderme en particulier.

D'autres études réalisées par les inventeurs de la présente invention ont permis également de mettre en évidence l'effet sur les aquaporines AQP3.

Ainsi, selon un autre aspect, l'invention concerne l'utilisation des extraits de l'invention ainsi que des associations telles que définies précédemment comme agent cosmétique régulateur, dans l'épiderme, des flux hydriques et de la ré-absorption d'eau.

Selon encore un autre aspect, l'invention concerne l'utilisation de ces extraits ou associations comme agent cosmétique destiné à hydrater l'épiderme.

L'invention concerne également selon un cinquième aspect, une composition cosmétique, notamment à activité de différenciation des kératinocytes, améliorant la fonction de la barrière hydrique de l'épiderme. à effet hydratant, à effet anti-vieillissement, ladite composition étant destinée à améliorer l'aspect et la qualité de la peau et à améliorer son hydratation par régulation, dans l'épiderme, des flux hydriques et de la ré-absorption d'eau, caractérisée en ce qu'elle contient une quantité cosmétiquement efficace pour le but recherché d'un extrait de la plante Ajuga turkestanica tel que défini précédemment ou d'une association telle que définie précédemment.

La teneur en extrait de la plante Ajuga turkestanica est de préférence comprise entre 0,005 et 10 %, de préférence entre 0,01 et 2 % en poids par rapport au poids total de la composition.

Par ailleurs, l'extrait de la plante Ajuga turkestanica selon la présente invention est avantageusement utilisé dans les compositions de l'invention en combinaison avec tout autre principe actif connu de l'homme de l'art, en particulier avec tout autre principe actif choisi dans le groupe constitué de la vitamine A et ses esters, en particulier le palmitate et l'acétate de vitamine A, la vitamine E et ses esters, en particulier le phosphate et l'acétate de vitamine E, la vitamine C et ses dérivés, en particulier l'ascorbyle phosphate de magnésium, les xanthines, en particulier la caféine, l'acide asiatique, l'acide madécassique, les asiaticosides, lés madécassosides, les extraits de Centella asiatica, l'acide ellagique, les saponines de soja, les extraits de Bertholletia, les extraits de Panax Ginseng, les eaux de source marine, l'aspartate de magnésium, le chlorure de manganèse, l'AMP cyclique, le D-xylose, l'acide hyaluronique, le gluconate de calcium, les isoflavones, le glycyrrhizinate d'ammonium, les corticostéroides, les extraits de Phellodendron amurense, le resvératrol et les picéides.

Les isoflavones citées ci-dessus sont notamment celles commercialisées sous le nom de "fujiflavones" par la société japonaise FUJICCO.

D'une façon générale, les compositions cosmétiques de l'invention contiennent des excipients ou supports cosmétiquement acceptables pour une application topique sur la peau. Des exemples de tels excipients ou supports cosmétiquement acceptables sont bien connus de l'homme de l'art.

Les compositions de l'invention peuvent être formulées sous toute forme cosmétiquement acceptable, en particulier sous forme de crème, de gel, de lotion.

L'invention concerne également un procédé de traitement cosmétique de la peau selon lequel on applique sur la peau une quantité cosmétiquement efficace d'extrait selon l'invention ou d'une association telle que définie précédemment.

On comprend que l'invention permet ainsi de mettre en oeuvre les propriétés particulièrement inattendues observées pour l'extrait de la plante Ajuga turkestanica.

D'autres buts, caractéristiques et avantages de l'invention apparaîtront clairement à l'homme de l'art à partir de la description explicative qui va suivre faite en référence à un test d'activité d'amélioration de la différenciation des kératinocytes ainsi qu'à un test mettant en évidence les transports hydriques dans l'épiderme. Divers exemples de formulations de compositions cosmétiques sont également donnés simplement à titre d'illustration et qui ne sauraient donc en aucune façon limiter la portée de l'invention. Dans les exemples, tous les pourcentages sont donnés en poids, sauf indication contraire.

### EXEMPLES

### A - Préparation d'extrait selon l'invention

On réalise une extraction à chaud de racines d'Ajuga turkestanica par un solvant composé pour 80% d'éthanol et pour 20% d'eau.

10 kg de matière sèche (racines d'Ajuga turkestanica) sont affrontés à 160 kg de solvant (mélange éthanol - eau dans les proportions respectives 80/20 ) à 50°C pendant 2 heures.

L'ensemble est refroidi jusqu'à température ambiante (environ 21°C).

L'ensemble refroidi est ensuite filtré sur un filtre 5µm (afin d'éliminer les matières en suspension et les matières ayant précipité).

L'ensemble filtré est évaporé à sec (pour éliminer le solvant d'extraction).

L'ensemble qui a été évaporé à sec est repris dans 1 litre d'eau.

L'ensemble qui a été repris dans 1 litre d'eau subit une homogénéisation forte (par agitation) plus des ultrasons. Ce traitement (homogénéisation + ultrasons) est poursuivi jusqu'à l'obtention d'une solution homogène.

L'ensemble homogénéisé subit une congélation rapide à -40°C et est maintenu à cette température pendant environ 4 heures.

L'ensemble congelé subit une lyophilisation.

Après lyophilisation, on obtient une poudre qui est un extrait sec d'Ajuga turkestanica selon l'invention, que nous nommerons " l'extrait S ".

Cet extrait a été analysé en recourant aux techniques analytiques classiques, notamment :
- utilisation de révélateurs spécifiques de chimie réactionnelle,
- chromatographie liquide haute performance (CLHP),
- chromatographie sur couches minces haute performance (CCMHP),
- chromatographie en phase gazeuse (CPG),
et répond à la composition donnée ci-dessous en pourcentage en poids :
ecdystérone 2,75 %
turkestérone 2,00 %
22-acétyl-cyastérone 1,93 %
2,3-monoacétonide d'ecdystérone 1,18 %
alpha-ecdysone 0,59 %
cyastérone 0,27 %
ajugastérone B 0,028 %
ajugalactone 0,016 %
8-O-acétyl-harpagide 16,70 %
harpagide 9,20 %
substances pectiniques 24,30 %
tannins 4,92 %
eau 6,95 %
monosaccharides (fructose, glucose) 19,98 %
cendres 6,97 %.

Cet extrait peut être incorporé dans des compositions cosmétiques.

Il est bien entendu possible d'améliorer cet extrait en lui faisant subir des purifications en vue, par exemple, d'éliminer les formes tanniques, les formes polysaccharidiques, les formes polypeptidiques et les pigments chlorophylliens. L'homme du métier dispose de nombreuses techniques pour réaliser ces purifications, nous citerons par exemple l'utilisation d'absorbeurs sélectifs tels que le charbon actif ou les gels de silice, les techniques de précipitation différentielles, les techniques de solubilisation différentielles ou les techniques de partition liquide-liquide.

Pour les besoins des exemples B, C et D suivants, cette poudre (" l'extrait S ") est remise en solution à raison de 25% de poudre, 25% d'eau et 50% d'éthanol, en poids total de la solution finale. Nous nommerons la solution obtenue " l'extrait L ".

### B - Mise en évidence de l'activité de l'extrait de l'invention sur la différenciation des kératinocytes humains normaux

Pour réaliser cet essai, on utilise l'extrait de racine de la plante Ajuga turkestanica " extrait L " obtenu selon l'exemple A.

Le présent essai utilise cet extrait pour mesurer la différenciation des kératinocytes humains normaux en culture cellulaire émergée.

Dans le cadre de l'invention, on entend par différenciation des kératinocytes l'ensemble des phénomènes impliqués dans la maturation des kératinocytes en coméocytes.

Pour ce faire, des cultures cellulaires émergées de kératinocytes humains traités par l'extrait selon l'invention et des cultures non traitées ont été comparées selon des critères morphologiques par des techniques de microscopie optique ou électronique.

Les conditions plus particulières de l'essai sont maintenant décrites ci-après.

### a) Obtention des cultures de kératinocytes humains normaux, dénommés en abrégé KHN

Des kératinocytes humains normaux ou KHN ont été obtenus à partir de peau de plastie mammaire d'une personne de race blanche de 29 ans, selon la méthode décrite par Eisinger M. dans un article ayant pour titre "Cultivation of normal human epidermal keratinocytes and melanocytes", dans le livre "Methods in skin research" paru aux éditions D. SKERROW and C.J. SKERROW, 1985, p. 191-212. Ces KHN, cultivés en milieu SFMc de chez Gibco, ont été utilisés pour la réalisation d'épidermes reconstruits sur lesquels l'extrait selon l'invention est testé.

### b) Réalisation des épidermes reconstruits

La culture émergée en insert est une technique décrite dans la littérature, en particulier par M.S. NOEL-HUDSON et al. dans l'article "Human epidermis reconstructed on synthetic membrane : Influence of experimental conditions on terminal differentiation", dans la revue In Vitro Cell. Dev. Biol., 1995, 31, p. 508-515.

Les cultures sont réalisées dans un système à deux compartiments (supérieur/inférieur) à l'aide de plaques COSTAR contenant 12 puits commercialisées par D. DUTSCHER à l'intérieur desquels sont déposés des inserts Transwell-clear COSTAR également commercialisés par DUTSCHER de diamètre 12 mm.

Les KHN sont ensemencés sur Transwell-clear, à raison de 115 000 KHN par insert, et cultivés en immersion dans du milieu SFMc jusqu'à confluence, le milieu de culture étant ajouté dans le compartiment supérieur.

A confluence des KHN, le milieu SFMc est ôté du compartiment supérieur, et le milieu de différenciation est alors additionné de l'extrait de la plante Ajuga turkestanica selon la présente invention et ensuite ajouté au compartiment inférieur, de façon à obtenir une culture à l'interface air/liquide, en émersion.

Le milieu de différenciation présente la composition essentielle suivante :
DMEM : HAM's F12 de chez Gibco contenant 5 % de sérum de veau foetal également de chez Gibco.

L'extrait selon l'invention (" extrait L " décrit à l'exemple A) a été conditionné en solution mère à 25 mg/ml dans le DMSO (diméthyl sulfoxyde), et filtré sur des filtres de porosité 0,22 µm, filtre Millex FGS, à capacité stérilisante. Le produit est ensuite mis au contact des cultures dès leur émersion, à 2,5, 10 et 25 µg/ml dans le milieu de différenciation, pendant 14 jours, avec renouvellement toutes les 48-72 h. Trois essais ont été réalisés pour chaque concentration testée.

Les cultures témoins ont reçu la même proportion de DMSO que les cultures traitées, soit 0,1 % v/v et le rythme de changement de milieu ainsi que la durée du traitement sont identiques à ceux des cultures traitées.

### c) Traitement des cultures pour l'analyse en microscopie

Après 14 jours de traitement avec l'extrait selon l'invention (" extrait L "), les épidermes reconstruits sont traités pour réaliser des observations en microscopie électronique et optique, qui permettent d'effectuer une étude ultrastructurale de la différenciation des kératinocytes de ces épidermes reconstruits.

Pour ce faire, les cultures cellulaires émergées subissent successivement une fixation tissulaire au glutaraldéhyde, une déshydratation à l'éthanol et une inclusion en résine, par exemple selon la méthode décrite par A.R. SPURR dans J. Ultrastruct. Res. USA, 1969, 26, p. 31-43, ayant pour titre "A low-viscosity epoxy resin embedding médium for électron microscopy".

On réalise ensuite des coupes semi-fines pour l'observation en microscopie optique et des coupes ultra-fines pour une observation en microscopie électronique.

### d) Observations

Les coupes semi-fines colorées au bleu de toluidine en microscopie optique, ont permis de mettre en évidence que l'épaisseur des couches de coméocytes constitutives de la couche cornée -ou stratum comeum- de l'épiderme, a augmenté très considérablement dans les épidermes reconstitués traités par l'extrait selon l'invention, par rapport aux épidermes reconstitués témoins. Cette augmentation est d'environ un facteur 3 à 4 selon les cultures.

En réalité, on a pu observer que le stratum comeum des cultures cellulaires émergées témoins était très réduit, tandis que, grâce au traitement au moyen de l'extrait de la plante Ajuga turkestanica selon l'invention, il a été possible de restaurer une couche cornée d'épaisseur normale.

L'épaisseur totale de la culture étant la même dans les deux cas, on peut observer qu'il existe plus de couches de kératinocytes basaux et supra-basaux pour la culture témoin. Il y a ici un effet de l'extrait de l'invention sur la différenciation kératinocytaire. C'est à l'endroit précis de l'interface entre la dernière couche supra-basale de kératinocyte et la première couche de coméocyte de stratum comeum que sont ensuite réalisées en microscopie électronique à transmission les observations qui sont ensuite rapportées ci-après.

D'autre part, pour les cultures cellulaires émergées traitées avec l'extrait selon l'invention aux doses de 10 µg/ml et 25 µg/ml, on observe le même type de profil de différenciation kératinocytaire, ce qui montre bien une uniformité entre toutes les cultures traitées par rapport à la culture témoin qui est vraiment très différente. En outre, en ce qui concerne les observations en microscopie électronique à transmission au niveau de l'interface entre les couches supra-basales (KH) et le stratum comeum (CO), à partir des coupes ultra-fines contrastées à l'acétate d'uranyl et au citrate de plomb, on peut juger de l'avancée de la différenciation kératinocytaire de la manière suivante :
a) Culture cellulaire émergée témoin, à agrandissement 30 000 :
   - desmosomes peu nombreux
   - desmosomes faiblement différenciés
   - enveloppe cornée peu visible
   - cornéocytes épais
   - réseau de kératine diffus
   - pas de coméodesmosome visible.
b) Pour les cultures cellulaires émergées traitées avec l'extrait selon l'invention respectivement aux doses de 2,5, 10, 25 µg/ml, les mêmes observations suivantes peuvent être réalisées :
   - desmosomes nombreux
   - desmosomes très bien différenciés
   - filaments de kératine reliés à la plaque desmosomale
   - filaments de kératine orientés parallèlement au coméocyte
   - enveloppe cornée bien visible du côté KH
   - coméocytes peu épais
   - réseau de kératine compact
   - présence de coméodesmosomes
   - espace intercoméocytaire observable (lipide).

En conclusion de ces essais, il résulte que les extraits selon l'invention apparaissent extrêmement actifs à faible dose. Dans les essais réalisés, l'extrait selon l'invention agit dès la concentration de 2,5 µg/ml sur la cohésion intercellulaire spécifiquement au niveau d'une des couches clé de l'épiderme à l'interface entre la couche cornée et les couches basales.

Par son caractère original, cet extrait optimise l'accroche entre les couches vivantes et la couche cornée, ce qui n'avait pas été observé jusqu'à présent in vitro.

Avec l'extrait selon l'invention, les filaments de kératine sont mieux organisés et accrochés aux plaques desmosomales. Ceci laisse fortement penser que la couche cornée peut subir des déformations locales liées aux contraintes physiques qu'elle reçoit sans subir de dommage grâce à une excellente élasticité locale. De plus, l'effet général de l'extrait de l'invention sur la formation d'une couche cornée de bonne qualité, grâce à l'obtention de cornéocytes peu épais avec une enveloppe cornée bien visible et un réseau de kératine compact, permet d'envisager une action régulatrice sur l'hydratation des couches superficielles de l'épiderme.

Ainsi, les extraits de l'invention permettront de renforcer le rôle de barrière hydrique de l'épiderme, d'améliorer l'état de l'épiderme et donc l'aspect et la qualité de la peau qui apparaîtra ainsi belle et douce. Grâce à son effet de différenciation des kératinocytes, l'invention permet d'obtenir un effet anti-vieillissement particulièrement intéressant.

### C - Mise en évidence de l'effet d'un extrait de l'invention sur le transport d'eau dans la peau

Les cultures de kératinocytes sont réalisées à partir de kératinocytes au deuxième passage issus d'une plastie chirurgicale effectuée sur une femme de 21 ans sur une membrane Costar Transwell de porosité 3 µm dans un milieu de différenciation pour kératinocytes tel que décrit par M.S. NOEL-HUDSON et al. dans l'article "Human epidermis reconstructed on synthetic membrane : Influence of experimental conditions on terminal differentiation", dans la revue In Vitro Cell. Dev. Biol., 1995, 31, p. 508-515.

Les cellules ont été traitées avec l'extrait selon l'invention (" Extrait L " tel que décrit dans l'exemple A) à la concentration de 2,5 µg/ml pendant 6 et 17 jours avec renouvellement du milieu tous les 2-3 Jours. L'extrait selon l'invention étant solubilisé dans le DMSO à 10 mg/ml et dilué extemporanément au 1/4000^{ème}. Les cellules témoins reçoivent la même quantité de DMSO.

On entend par J0 le jour où les cultures ayant atteint la confluence sont émergées de leur milieu de culture et exposées à l'air.

Les mesures de perméabilité ont été réalisées à J=0 ; J=6 et J=17 selon un protocole décrit par R. A. Dorr et al. dans "A new data-acquisition system for the measurement of the net water flux across epithelia" ; Computer Methods and Programs in Biomedicine 1997, 53, pp 9-14.

Pour réaliser les mesures de perméabilité, on dispose les filtres dans une chambre de mesure composée de deux compartiments remplis de milieu de culture. Le compartiment luminal (partie supérieure de la culture) est fermé par un cathéter relié à un tube capillaire horizontal rempli de colorant. Tout mouvement de liquide se traduit par un déplacement du colorant que l'on peut mesurer. Le flux d'eau sera mesuré en absence de gradient osmotiqué (isotonicité dans les deux milieux). Le flux d'eau mesuré est la conséquence d'un flux net de soluté à travers la préparation. La perméabilité hydrique se mesure en µl/min/cm².

A J=0 les cellules ne présentent pas de barrière significative au passage de l'eau démontré par une absorption massive d'eau lors de l'application d'une pression hydrostatique dans le compartiment luminal. A partir de J0, les cellules se différencient progressivement en multicouches et à partir de J4 ou J7 selon les souches utilisées, les cellules développent une résistance au passage de l'eau.

Le tableau ci-dessous donne les flux d'eau dans des épidermes humains de cultures traitées avec l'extrait selon l'invention (" Extrait L " tel que décrit dans l'exemple A) à la concentration de 2,5 µg/ml. Les flux sont exprimés en µl/min/cm².

| Temps de traitement en jours | Epidermes témoins µl/min/cm² | Epidermes traités µl/min/cm² |
|---|---|---|
| J6 | - 0,49 | 0,76 |
| J17 | - 0,015 | 0,12 |

Une valeur négative indique une sécrétion, une valeur positive un flux d'absorption. Contrairement aux cellules témoins pour lesquelles une sécrétion d'eau est mesurée, les cellules traitées par l'extrait d'Ajuga turkestanica selon l'invention absorbent l'eau de manière importante. L'importance de cette absorption décroît à j= 17 mais reste très significative. La raison de cette baisse résulte de l'augmentation de l'épaisseur des épidermes et de la formation du stratum corneum et de son imperméabilité à l'eau. Les flux d'eau sont donc moindre. Ceci est également vrai pour les cultures témoins mais dans une moindre mesure.

### CONCLUSION :

La présente étude démontre que le produit décrit dans l'exemple A (" Extrait L ") utilisé à la concentration de 2,5 µg/ml dans les conditions précitées favorise fortement un phénomène de ré-absorption d'eau. Le flux va vers les couches germinatives kératinocytaires et favorise leur hydratation. Cette ré-absorption d'eau permet d'augmenter ou de maintenir l'hydratation des couches "vivantes " (kératinocytes) de l'épiderme localisées sous la couche cornée de l'épiderme.

### D - Mise en évidence de l'action d'un extrait de l'invention sur la fonctionnalité des aquaporines AQP3

R. SOUGRAT et al. dans un article "Functional expression of AQP3 in human epidermis and keratinocyte cell cultures" publié dans Molecular Biology of the Cell ; Nov. 1998 ; vol. 9, page 499a, a décrit la présence d'aquaporines 3 (AQP3) dans la membrane plasmique des kératinocytes de la peau humaine. Il décrit également leur rôle important dans le transport de l'eau au sein de l'épiderme humain.

On a montré dans l'exemple C que l'extrait selon l'invention augmente fortement les flux de ré-absorption de l'eau dans l'épiderme humain. Compte tenu de l'importance des flux d'eau mesurés, ceux-ci ne peuvent s'expliquer que par un transport de l'eau contrôlé par les AQP3. Un passage transcellulaire au travers de la bicouche phospholipidique de la membrane plasmique des kératinocytes serait de beaucoup plus faible amplitude. Un contrôle a permis d'écarter la possibilité d'un passage péri-cellulaire de l'eau. En effet, lorsque l'on fait varier la pression hydrostatique appliquée sur l'épiderme, le flux d'eau n'est pas affecté.

C'est pourquoi les inventeurs de la présente invention en ont déduit que ces extraits étaient particulièrement actifs pour réguler et/ou améliorer la fonctionnalité des aquaporines 3 (AQP3).

### E - Compositions cosmétiques

Dans les compositions cosmétiques décrites ci-dessous, les extraits d'Ajuga turkestanica sont des extraits secs, sauf indication contraire.

### E1 - Emulsion hydratante et nourrisante anti-âge

| | |
|---|---|
| Extrait d'Ajuga turkestanica (extrait S) | 0,025 g |
| Acétate de vitamine E | 0,100 g |
| Palmitate de vitamine A | 0,010 g |
| Ascorbyle phosphate de magnésium | 0,200 g |
| Céramides de blé | 0,200 g |
| Protéines de blé | 1,000 g |
| Filtre UVA-UVB | 5,000 g |
| Excipient avec conservateurs et parfums | qsp. 100g |

L'extrait d'Ajuga turkestanica (extrait S) utilisé dans cette Emulsion est l'extrait décrit ci-dessus dans l'exemple A.

Cette émulsion nourrissante améliore l'hydratation cutanée en particulier de l'épidémie, la finesse et le grain de la peau et atténue les rides. Elle est appliquée quotidiennement sur le visage.

### E2 - Gel hydratant raffermissant et restructurant

| | |
|---|---|
| Extrait d'Ajuga turkestanica (extrait S) | 0,040 g |
| Aspartate de magnésium | 0,100 g |
| Extrait de Centella asiatica | 0,100 g |
| Saponines de soja | 0,050 g |
| Excipient avec conservateurs et parfums | qsp. 100g |

L'extrait d'Ajuga turkestanica (extrait S) utilisé dans ce gel est l'extrait décrit ci-dessus dans l'exemple A.

Ce gel hydratant exerce un effet tenseur sur la peau et améliore la souplesse et la fermeté. Il est appliqué quotidiennement sur le visage, le cou et le buste.

### E3 - Gel liposomal hydratant réparateur

| | |
|---|---|
| Extrait d'Ajuga turkestanica (extrait S) | 0,020 g |
| Lecithine de soja | 2,000 g |
| Acétate de vitamine A | 0,010 g |
| Vitamine E | 0,010 g |
| Excipient avec conservateurs et parfums | qsp. 100g |

L'extrait d'Ajuga turkestanica (extrait S) utilisé dans ce gel est l'extrait décrit ci-dessus dans l'exemple A.

Ce gel liposomal est utilisé en application quotidienne, de préférence le soir et sur le visage. Il améliore la souplesse de la peau et son hydratation. Il favorise sa régénération.

### E4 - Lotion hydratante et tonifiante

| | |
|---|---|
| Extrait d'Ajuga turkestanica (extrait S) | 0,030 g |
| Eau de source marine concentrée en oligoéléments | 10,000 g |
| Extrait de Panax Ginseng | 0,200 g |
| AMP cyclique | 0,010 g |
| Caféine | 0,100 g |
| Excipient avec conservateurs et parfums | qsp. 100g |

L'extrait d'Ajuga turkestanica (extrait S) utilisé dans cette lotion est l'extrait décrit ci-dessus dans l'exemple A.

Cette lotion hydratante est utilisée en application quotidienne sur le visage, de préférence le matin pour obtenir une peau plus belle, plus éclatante.

### E5 - Fluide hydratant et apaisant

| | |
|---|---|
| Extrait d'Ajuga turkestanica | 0.04 g |
| Chlorure de manganèse | 0,10 g |
| D-xylose | 0,10 g |
| Céramides de blé | 0,20 g |
| Extrait de réglisse | 0,10 g |
| Acétate de vitamine E | 0.20 g |
| Excipient avec conservateurs et parfums | qsp. 100g |

L'extrait d'Ajuga turkestanica utilisé dans ce fluide est un extrait obtenu avec un solvant composé pour 80% de méthanol et pour 20% d'eau. Le procédé d'extraction est similaire à celui décrit dans l'exemple A, à l'exception de la nature du mélange de solvants.

Ce fluide est utilisé en application topique quotidienne sur le visage et le corps.

### E6 - Mascara hydratant

| | |
|---|---|
| Extrait d'Ajuga turkestanica | 0,025 g |
| Acide hyaluronique | 0,500 g |
| D-xylose | 0.200 g |
| Pigments colorés | 10.000 g |
| Cires | 30,000 g |
| Excipient avec conservateurs et parfums | qsp. 100g |

L'extrait d'Ajuga turkestanica utilisé dans ce mascara est un extrait obtenu avec un solvant composé pour 80% de acétone et pour 20% d'eau. Le procédé d'extraction est similaire à celui décrit dans l'exemple A, à l'exception de la nature du mélange de solvants.

### E7 - Patch ré-hydratant et anti-vieillissement après soleil

| | |
|---|---|
| Extrait d'Ajuga turkestanica | 0,5 g |
| Glycyrrhizinate d'ammonium | 0.5 g |
| Dextran sulfate | 0,2 g |
| Excipient pour patch | qsp. 100g |

L'extrait d'Ajuga turkestanica utilisé dans ce patch est un extrait obtenu avec un solvant composé pour 50% de propylène glycol et pour 50% d'eau. Le procédé d'extraction est similaire à celui décrit dans l'exemple A, à l'exception de la nature du mélange de solvants.

### E8 - Crème antiseptique hydratante pour les gerçures, petites plaies superficielles et rougeurs d'irritation

| | |
|---|---|
| Extrait d'Ajuga turkestanica | 0,4 g |
| Extrait de Phellodendron amurense | 0,1 g |
| Acide fusidique | 2,0 g |
| Excipient | qsp. 100g |

L'extrait d'Ajuga turkestanica utilisé dans cette crème est un extrait obtenu avec un solvant composé pour 50% de butylène glycol et pour 50% d'eau. Le procédé d'extraction est similaire à celui décrit dans l'exemple A, à l'exception de la nature du mélange de solvants.

### E9 - Emulsion hydratante et nourrissante anti-âge

| | |
|---|---|
| Ecdystérone | 0,007 g |
| Turkestérone | 0,005 g |
| 22-acétyl-cyastérone | 0,005 g |
| 2,3-monoacétonide d'ecdystérone | 0.003 g |
| Alpha-ecdysone | 0,0015 g |
| Cyastérone | 0,0007 g |
| 8-O-acétyl-harpagide | 0.04 g |
| Harpagide | 0,02 g |
| Acétate de vitamine E | 0,100 g |
| Palmitate de vitamine A | 0,010 g |
| Ascorbyle phosphate de magnésium | 0,200 g |
| Céramides de blé | 0.200 g |
| Protéines de blé | 1.000 g |
| Filtre UVA-UVB | 5,000 g |
| Excipient avec conservateurs et parfums | qsp. 100g |

## Revendications

1. Extrait de la plante Ajuga turkestanica contenant au moins un ecdystéroïde et au moins un iridoïde, **caractérisé en ce qu'**il contient une partie en poids d'au moins un ecdystéroïde et 2 à 4 parties en poids, de préférence 3 parties en poids, d'au moins un iridoïde.

2. Extrait selon la revendication 1, **caractérisé en ce que** ledit ecdystéroïde est choisi dans le groupe constitué par l'ecdystérone, le 2,3-moaoacétonide d'ecdystérone, la turkestérone, la cyastérone, la 22-acétyl-cyastérone et l'alpha-ecdysone.

3. Extrait selon la revendication 1 ou 2, **caractérisé en ce que** ledit iridoïde est choisi dans le groupe constitué par l'harpagide et son dérivé 8-O-acétylé.

4. Extrait selon l'une des revendications 1 à 3, **caractérisé en ce que** lesdits ecdystéroïdes contenus dans ledit extrait sont composés, exprimé en poids, de 30 à 35 % d'ecdystérone, 20 à 25 % de turkestérone, 20 à 24 % de 22-acétyl-cyastérone, 11 à 15 % de 2,3-monoacétonide d'ecdystérone, 6 à 7 % d'alpha-ecdysone, 2 à 4 % de cyastérone.

5. Extrait selon l'une des revendications 1 à 4, **caractérise en ce que** lesdits iridoïdes contenus dans ledit extrait sont composés, exprimé en poids, de 60 à 70 % de 8-O-acétyl-harpagide et de 30 à 40 % d'harpagide.

6. Extrait selon l'une des revendications 1 à 5, **caractérisé en ce qu'**il est susceptible d'être obtenu par extraction d'une partie au moins de ladite plante au moyen d'un solvant ou d'un mélange de solvants constitué de 0 à 60 % en poids d'eau, le reste dudit solvant ou mélange de solvants étant constitué d'au moins un alcool en C₁ à C₄, et/ou de l'acétone et/ou du butylène glycol et/ou du propylène glycol.

7. Extrait selon la revendication 6, **caractérisé en ce que** ladite extraction est réalisée avec ledit solvant ou mélange de solvants pendant 1 à 4 heures.

8. Extrait selon la revendication 6, **caractérisé en ce que** ladite extraction est réalisée au reflux dudit solvant ou mélange de solvants pendant 1 à 4 heures.

9. Extrait selon l'une des revendications 6 à 8, **caractérisé en ce que** ladite extraction est réalisée à partir de ladite plante entière.

10. Extrait selon l'une des revendications 6 à 8, **caractérisé en ce que** ladite extraction est réalisée à partir des racines de ladite plante.

11. Extrait selon l'une des revendications 6 à 10, **caractérisé en ce que** ledit solvant ou mélange de solvants est un mélange d'eau et d'éthanol.

12. Extrait selon la revendication 11, **caractérisé en ce que** le mélange d'eau et d'éthanol est un mélange contenant 70 à 90 % d'éthanol et 30 à 10 % d'eau, de préférence 80 % d'éthanol pour 20 % d'eau.

13. Extrait selon l'une des revendications 6 à 10, **caractérisé en ce que** ledit solvant ou mélange de solvants est un mélange d'eau et de méthanol.

14. Extrait selon la revendication 13, **caractérisé en ce que** ledit mélange d'eau et de méthanol est un mélange contenant 70 à 90 % de méthanol et 30 à 10 % d'eau, de préférence 80 % de méthanol pour 20 % d'eau.

15. Extrait selon l'une des revendications 6 à 10, **caractérisé en ce que** ledit solvant ou mélange de solvants est un mélange d'eau et d'acétone.

16. Extrait selon la revendication 15 **caractérisé en ce que** le mélange d'eau et d'acétone est un mélange contenant 70 à 90 % d'acétone et 30 à 10 % d'eau, de préférence 80 % d'acétone pour 20 % d'eau.

17. Extrait selon l'une des revendications 6 à 10, **caractérisé en ce que** ledit solvant ou mélange de solvants est un mélange d'eau et de propylène glycol.

18. Extrait selon la revendication 17 **caractérisé en ce que** le mélange d'eau et de propylène glycol est un mélange contenant 40 à 60 % de propylène glycol et 60 à 40 % d'eau, de préférence 50 % de propylène glycol pour 50 % d'eau.

19. Extrait selon l'une des revendications 6 à 10, **caractérisé en ce que** ledit solvant ou mélange de solvants est un mélange d'eau et de butylène glycol.

20. Extrait selon la revendication 19, **caractérisé en ce que** le mélange d'eau et de butylène glycol est un mélange contenant 40 à 60 % de butylène glycol et 60 à 40 % d'eau, de préférence 50 % de butylène glycol pour 50 % d'eau.

21. Composition cosmétique, notamment à activité de différenciation des kératinocytes, améliorant la fonction de la barrière hydrique de l'épiderme, à effet hydratant, à effet anti-vieillissement, ladite composition étant destinée à améliorer l'aspect et la qualité de la peau et à améliorer son bydratation par régulation dans l'épiderme des flux hydriques et de la ré-absorption d'eau, **caractérisée en ce qu'**elle contient une quantité cosmétiquement efficace pour le but recherché d'un extrait de la plante Ajuga turkestanica tel que défini dans l'une des revendications 1 à 20,

22. Composition cosmétique, notamment à activité de différenciation des kératinocytes, améliorant la fonction de la barrière hydrique de l'épiderme, à effet hydratant, à effet anti-vieillissement, ladite composition étant destinée à améliorer l'aspect et la qualité de la peau et à améliorer son hydratation par régulation dans l'épiderme des flux hydriques et de la ré-absorption d'eau, **caractérisée en ce qu'**elle contient une quantité cosmétiquement efficace pour le but recherché d'une association comprenant une partie en poids d'au moins un ecdystéroïde et 2 à 4 parties en poids, de préférence 3 parties en poids, d'au moins un iridoïde.

23. Composition selon la revendication 22, **caractérisée en ce que** ledit ecdystéroïde est choisi dans le groupe constitué par l'ecdystérone, le 2,3-monoacétonide d'ecdystérone, la turkestérone, la cyastérone, la 22-acétyl-cyastérone et l'alpha-ecdysone.

24. Composition selon la revendication 22 ou 23, **caractérisée en ce que** ledit iridoïde est choisi dans le groupe constitué par l'harpagide et son dérivé 8-O-acétylé.

25. Composition selon l'une des revendications 22 à 24, **caractérisée en ce que** les ecdystéroïdes présents dans ladite association sont composés, exprimé en poids, de 30 à 35 % d'ecdystérone, 20 à 25 % de turkestérone, 20 à 24 % de 22-acétyl-cyastérone, 11 à 15 % de 2,3-monoacétonide d'ecdystérone, 6 à 7 % d'alpha-ecdysone, 2 à 4 % de cyastérone.

26. Composition selon l'une des revendications 22 à 25, **caractérisée en ce que** les iridoïdes présents dans ladite association sont composés, exprimé en poids, de 60 à 70 % de 8-O-acécyl-harpagide et de 30 à 40 % d'harpagide.

27. Composition selon l'une des revendications 21 à 26, **caractérisée en ce qu'**elle contient de 0,005 à 10 %, de préférence de 0,01 à 2 % en poids par rapport au poids total de ladite composition d'un extrait tel que défini dans l'une des revendications 1 à 20 ou d'une association telle que définie dans l'une des revendications 22 à 26.

28. Composition selon l'une des revendications 21 à 27, **caractérisée en ce qu'**elle contient en outre au moins une substance choisie dans le groupe constitué de la vitamine A et ses esters en particulier le palmitate et l'acétate de vitamine A, la vitamine E et ses esters, en particulier le phosphate et l'acétate de vitamine E, la vitamine C et ses dérivés, en particulier l'ascorbyle phosphate de magnésium, les xanthines, en particulier la caféine, l'acide asiatique, l'acide madecassique, les asiaticosides, les madécassosides, les extraits de Centella asiatica, l'acide ellagique, les saponines de soja, les extraits de Bertholletia, les extraits de Panax Ginseng, les eaux de source marine, l'aspartate de magnésium, le chlorure de manganèse, l'AMP cyclique, le D-xylose, l'acide hyaluronique, le gluconate de calcium, les isoflavones, le glycyrrhizinate d'ammonium, les corticostéroïdes, les extraits de Phellodendron amurense, le resvératrol et les picéides.

29. Utilisation non thérapeutique d'un extrait selon l'une des revendications 1 à 20 ou d'une association telle que définie dans l'une des revendications 22 à 26, comme agent cosmétique destiné à améliorer la différenciation des kératinocytes.

30. Utilisation non thérapeutique d'un extrait selon l'une des revendications 1 à 20 ou d'une association telle que définie dans l'une des revendications 22 à 26, comme agent cosmétique régulateur, dans l'épiderme, des flux hydriques et de la ré-absorption d'eau.

31. Utilisation non thérapeutique d'un extrait selon l'une des revendications 1 à 20 ou d'une association telle que définie dans l'une des revendications 22 à 26, comme agent cosmétique destiné à hydrater l'épiderme.

32. Procédé de traitement cosmétique de la peau, **caractérisé en ce que** l'on applique sur la peau une quantité cosmétiquement efficace d'un extrait selon l'une des revendications 1 à 20 ou d'une association telle que définie dans l'une des revendications 22 à 26.

## Patentansprüche

1. Ajuga turkestania Extrakt, enthaltend mindestens ein Ecdysteroid und mindestens ein Iridoid, **dadurch gekennzeichnet, dass** er einen Gewichtsanteil von mindestens einem Ecdysteroid und 2 bis 4 Gewichtsanteile, vorzugsweise 3 Gewichtsanteile, von mindestens einem Iridoid enthält.

2. Extrakt nach Anspruch 1, **dadurch gekennzeichnet, dass** das Ecdysteroid ausgewählt ist aus der Gruppe, bestehend aus Ecdysteron, 2,3-Ecdysteron-Monoacetonid, Turkesteron, Cyasteron, 22-Acetylcyasteron und Alpha-Ecdyson.

3. Extrakt nach Anspruch 1 oder 2, **dadurch gekennzeichnet, dass** das Iridoid ausgewählt ist aus der Gruppe, bestehend aus Harpagid und dessen Derivat 8-O-Acetyl.

4. Extrakt nach einem der Ansprüche 1 bis 3, **dadurch gekennzeichnet, dass** die in dem Extrakt enthaltenen Ecdysteroide zusammengesetzt sind aus 30 bis 35 Gew.-% Ecdysteron, 20 bis 25 Gew.-% Turkesteron, 20 bis 24 % 22-Acetylcyasteron, 11 bis 15 Gew.-% 2,3-Ecdysteron-Monoacetonid, 6 bis 7 Gew.-% Alpha-Ecdyson, 2 bis 4 Gew.-% Cyasteron.

5. Extrakt nach einem der Ansprüche 1 bis 4, **dadurch gekennzeichnet, dass** die in dem Extrakt enthaltenen Iridoide zusammengesetzt sind aus 60 bis 70 Gew.-% 8-O-Acetyl-Harpagid und 30 bis 40 Gew.-% Harpagid.

6. Extrakt nach einem der Ansprüche 1 bis 5, **dadurch gekennzeichnet, dass** er hergestellt werden kann durch Extraktion aus mindestens einem Teil der Pflanze mit Hilfe eines Lösungsmittels oder eines Lösungsmittelgemischs, bestehend aus 0 bis 60 Gew.-% Wasser, wobei der Rest des Lösungsmittels oder Lösungsmittelgemischs aus mindestens einem Alkohol in C₁ bis C₄ und/oder Aceton und/oder Butylenglycol und/oder Propylenglycol.

7. Extrakt nach Anspruch 6, **dadurch gekennzeichnet, dass** die Extraktion mit dem Lösungsmittel oder Lösungsmittelgemisch während 1 bis 4 Stunden ausgeführt wird.

8. Extrakt nach Anspruch 6, **dadurch gekennzeichnet, dass** die Extraktion im Rückfluss des Lösungsmittels oder Lösungsmittelgemischs während 1 bis 4 Stunden ausgeführt wird.

9. Extrakt nach einem der Ansprüche 6 bis 8, **dadurch gekennzeichnet, dass** die Extraktion an der ganzen Pflanze ausgeführt wird.

10. Extrakt nach einem der Ansprüche 6 bis 8, **dadurch gekennzeichnet, dass** die Extraktion an den Wurzeln der Pflanze ausgeführt wird.

11. Extrakt nach einem der Ansprüche 6 bis 10, **dadurch gekennzeichnet, dass** das Lösungsmittel oder Lösungsmittelgemisch ein Gemisch aus Wasser und Ethanol ist.

12. Extrakt nach Anspruch 11, **dadurch gekennzeichnet, dass** das Gemisch aus Wasser und Ethanol ein Gemisch ist, das 70 bis 90 % Ethanol und 30 bis 10 % Wasser, vorzugsweise 80 % Ethanol für 20 % Wasser, enthält.

13. Extrakt nach einem der Ansprüche 6 bis 10, **dadurch gekennzeichnet, dass** das Lösungsmittel oder Lösungsmittelgemisch ein Gemisch aus Wasser und Methanol ist.

14. Extrakt nach Anspruch 13, **dadurch gekennzeichnet, dass** das Gemisch aus Wasser und Methanol ein Gemisch ist, das 70 bis 90 % Methanol und 30 bis 10 % Wasser, vorzugsweise 80 % Methanol für 20 % Wasser, enthält.

15. Extrakt nach einem der Ansprüche 6 bis 10, **dadurch gekennzeichnet, dass** das Lösungsmittel oder Lösungsmittelgemisch ein Gemisch aus Wasser und Aceton ist.

16. Extrakt nach Anspruch 15, **dadurch gekennzeichnet, dass** das Gemisch aus Wasser und Aceton ein Gemisch ist, das 70 bis 90 % Aceton und 30 bis 10 % Wasser, vorzugsweise 80 % Aceton für 20 % Wasser, enthält,

17. Extrakt nach einem der Ansprüche 6 bis 10, **dadurch gekennzeichnet, dass** das Lösungsmittel oder Lösungsmittelgemisch ein Gemisch aus Wasser und Propylenglycol ist.

18. Extrakt nach Anspruch 17, **dadurch gekennzeichnet, dass** das Gemisch aus Wasser und Propylenglycol ein Gemisch ist, das 40 bis 60 % Propylenglycol und 60 bis 40 % Wasser, vorzugsweise 50 % Propylenglycol für 50 % Wasser, enthält.

19. Extrakt nach einem der Ansprüche 6 bis 10, **dadurch gekennzeichnet, dass** das Lösungsmittel oder Lösungsmittelgemisch ein Gemisch aus Wasser und Butylenglycol ist.

20. Extrakt nach Anspruch 19, **dadurch gekennzeichnet, dass** das Gemisch aus Wasser und Butylenglycol ein Gemisch ist, das 40 bis 60 % Butylenglycol und 60 bis 40 % Wasser, vorzugsweise 50 % Propylenglycol für 50 % Wasser, enthält.

21. Kosmetische Zusammensetzung, insbesondere mit einer Wirkung zur Differenzierung von Keratinocyten, was die Funktion der Wasserbarriere der Epidermis verbessert, mit hydratierender Wirkung, mit alterungshemmender Wirkung, wobei die Zusammensetzung dazu bestimmt ist, das Aussehen und die Beschaffenheit der Haut zu verbessern und deren Hydratierung durch Regulierung der Wasserströme und die Wiederaufnahme von Wasser in der Epidermis zu verbessern, **dadurch gekennzeichnet, dass** sie eine für das angestrebte Ziel kosmetisch wirksame Menge eines Ajuga turkestanica Extrakts enthält, wie in einem der Ansprüche 1 bis 20 definiert.

22. Kosmetische Zusammensetzung, insbesondere mit einer Wirkung zur Differenzierung von Keratinocyten, was die Funktion der Wasserbarriere der Epidermis verbessert, mit hydratierender Wirkung, mit alterungshemmender Wirkung, wobei die Zusammensetzung dazu bestimmt ist, das Aussehen und die Beschaffenheit der Haut zu verbessern und deren Hydratierung durch Regulierung der Wasserströme und die Wiederaufnahme von Wasser in der Epidermis zu verbessern, **dadurch gekennzeichnet, dass** sie eine für das angestrebte Ziel kosmetisch wirksame Menge einer Verbindung enthält, die einen Gewichtsanteil von mindestens einem Ecdysteroid und 2 bis 4 Gewichtsanteile, vorzugsweise 3 Gewichtsanteile, von mindestens einem Iridoid umfasst.

23. Zusammensetzung nach Anspruch 22, **dadurch gekennzeichnet, dass** das Ecdysteroid ausgewählt ist aus der Gruppe bestehend aus Ecdysteron, 2,3-Ecdysteron-Monoacetonid, Turkesteron, Cyasteron, 22-Acetyl-Cyasteron und Alpha-Ecdyson.

24. Zusammensetzung nach Anspruch 22 oder 23, **dadurch gekennzeichnet, dass** das Iridoid ausgewählt ist aus der Gruppe bestehend aus Harpagid und seinem Derivat 8-O-Acetyl.

25. Zusammensetzung nach einem der Ansprüche 22 bis 24, **dadurch gekennzeichnet, dass** die in der Verbindung enthaltenen Ecdysteroide zusammengesetzt sind aus 30 bis 35 Gew.-% Ecdysteron, 20 bis 25 Gew.-% Turkesteron, 20 bis 24 Gew.-% 22-Acetyl-Cyasteron, 11 bis 15 Gew.-% 2,3-Ecdysteron-Monoacetonid, 6 bis 7 Gew.-% Alpha-Ecdysteron, 2 bis 4 Gew.-% Cyasteron.

26. Zusammensetzung nach einem der Ansprüche 22 bis 25, **dadurch gekennzeichnet, dass** die in der Verbindung vorhandenen Iridoide zusammengesetzt sind aus 60 bis 70 Gew.-% 8-O-Acetyl-Harpagid und 30 bis 40 Gew.-% Harpagid.

27. Zusammensetzung nach einem der Ansprüche 21 bis 26, **dadurch gekennzeichnet, dass** sie 0,005 bis 10 Gew.-%, vorzugsweise 0,01 bis 2 Gew.-%, in Bezug auf das Gesamtgewicht der Zusammensetzung eines Extrakts, wie er in einem der Ansprüche 1 bis 20 definiert ist, oder einer Verbindung, wie sie in einem der Ansprüche 22 bis 26 definiert ist, enthält.

28. Zusammensetzung nach einem der Ansprüche 21 bis 27, **dadurch gekennzeichnet, dass** sie außerdem mindestens eine Substanz enthält, ausgewählt aus der Gruppe bestehend aus Vitamin A und seinen Estern, insbesondere Vitamin-A-Palmitat und -Acetat, Vitamin E und seinen Estern, insbesondere Vitamin-E-Phosphat und -Acetat, Vitamin C und seinen Derivaten, insbesondere Magnesium-Ascorbylphosphat, Xanthinen, insbesondere Cafein, asiatischer Säure, madekassicher Säure, Asiaticosiden, Madekassosiden, Centella asiatica- Extrakten, Ellaginsäure, Sojasaponinen, Bertholletia-Extrakten, Panax-Ginseng-Extrakten, Meerquellwasser, Magnesiumaspartat, Manganchlorid, cyclischem AMP, D-Xylose, Hyaluronsäure, Calciumgluconat, Isoflavonen, Ammoniumglycyrrhizinat, Corticosteroiden, Phellodendron amurense-Extrakten, Resveratrol und Piceiden.

29. Nicht-therapeutische Verwendung eines Extrakts nach einem der Ansprüche 1 bis 20 oder einer Verbindung wie in einem der Ansprüche 22 bis 26 definiert als kosmetischer Wirkstoff zur Verbesserung der Differenzierung der Keratinocyten.

30. Nicht-therapeutische Verwendung eines Extrakts nach einem der Ansprüche 1 bis 20 oder einer Verbindung wie in einem der Ansprüche 22 bis 26 definiert als kosmetischer Wirkstoff zur Regulierung der Wasserströme und der Wiederaufnahme von Wasser in der Epidermis.

31. Nicht-therapeutische Verwendung eines Extrakts nach einem der Ansprüche 1 bis 20 oder einer Verbindung wie in einem der Ansprüche 22 bis 26 definiert, als kosmetischer Wirkstoff zur Hydratierung der Epidermis.

32. Verfahren zur kosmetischen Behandlung der Haut, **dadurch gekennzeichnet, dass** man auf die Haut eine kosmetisch wirksame Menge eines Extrakts nach einem der Ansprüche 1 bis 20 oder einer Verbindung wie in einem der Ansprüche 22 bis 26 definiert aufträgt.

## Claims

1. Extract of the plant Ajuga turkestanica containing at least one ecdysteroid and at least one iridoid, **characterized in that** it contains one part by weight of at least one ecdysteroid and 2 to 4 parts by weight, preferably 3 parts by weight, of at least one iridoid.

2. Extract according to claim 1, **characterized in that** said ecdysteroid is selected from the group consisting of ecdysterone, ecdysterone 2,3-monoacetonide, turkesterone, cyasterone, 22-acetylcyasterone and alpha-ecdysone.

3. Extract according to claim 1 or 2, **characterized in that** said iridoid is selected from the group consisting of harpagide and its 8-O-acetyl derivative.

4. Extract according to one of claims 1 to 3, **characterized in that** said ecdysteroids contained in said extract are composed of 30 to 35% of ecdysterone, 20 to 25% of turkesterone, 20 to 24% of 22-acetylcyasterone, 11 to 15% of ecdysterone 2,3-monoacetonide, 6 to 7% of alpha-ecdysone and 2 to 4% of cyasterone, the percentages being expressed by weight.

5. Extract according to one of claims 1 to 4, **characterized in that** said iridoids contained in said extract are composed of 60 to 70% of 8-O-acetylharpagide and 30 to 40% of harpagide, the percentages being expressed by weight.

6. Extract according to one of claims 1 to 5, **characterized in that** it is obtainable by extraction of at least part of said plant by means of a solvent or solvent mixture consisting of 0 to 60% by weight of water, the remainder of said solvent or solvent mixture consisting of at least one C₁ to C₄ alcohol and/or acetone and/or butylene glycol and/or propylene glycol.

7. Extract according to claim 6, **characterized in that** said extraction is performed with said solvent or solvent mixture for 1 to 4 hours.

8. Extract according to claim 6, **characterized in that** said extraction is performed at the reflux temperature of said solvent or solvent mixture for 1 to 4 hours.

9. Extract according to one of claims 6 to 8, **characterized in that** said extraction is performed on the whole of said plant.

10. Extract according to one of claims 6 to 8, **characterized in that** said extraction is performed on the roots of said plant.

11. Extract according to one of claims 6 to 10, **characterized in that** said solvent or solvent mixture is a mixture of water and ethanol.

12. Extract according to claim 11, **characterized in that** the mixture of water and ethanol is a mixture containing 70 to 90% of ethanol and 30 to 10% of water, preferably 80% of ethanol to 20% of water.

13. Extract according to one of claims 6 to 10, **characterized in that** said solvent or solvent mixture is a mixture of water and methanol.

14. Extract according to claim 13, **characterized in that** said mixture of water and methanol is a mixture containing 70 to 90% of methanol and 30 to 10% of water, preferably 80% of methanol to 20% of water.

15. Extract according to one of claims 6 to 10, **characterized in that** said solvent or solvent mixture is a mixture of water and acetone.

16. Extract according to claim 15, **characterized in that** the mixture of water and acetone is a mixture containing 70 to 90% of acetone and 30 to 10% of water, preferably 80% of acetone to 20% of water.

17. Extract according to one of claims 6 to 10, **characterized in that** said solvent or solvent mixture is a mixture of water and propylene glycol.

18. Extract according to claim 17, **characterized in that** the mixture of water and propylene glycol is a mixture containing 40 to 60% of propylene glycol and 60 to 40% of water, preferably 50% of propylene glycol to 50% of water.

19. Extract according to one of claims 6 to 10, **characterized in that** said solvent or solvent mixture is a mixture of water and butylene glycol.

20. Extract according to claim 19, **characterized in that** the mixture of water and butylene glycol is a mixture containing 40 to 60% of butylene glycol and 60 to 40% of water, preferably 50% of butylene glycol to 50% of water.

21. Cosmetic composition, especially with activity in the differentiation of keratinocytes, which improves the function of the epidermis as a water barrier and has a hydrating effect and an anti-ageing effect, said composition being intended to improve the appearance and quality of the skin and to improve its hydration by regulation of the water fluxes and the water absorption in the epidermis, **characterized in that** it contains a cosmetically effective amount, for the intended purpose, of an extract of the plant Ajuga turkestanica as defined in one of claims 1 to 20.

22. Cosmetic composition, especially with activity in the differentiation of keratinocytes, which improves the function of the epidermis as a water barrier and has a hydrating effect and an anti-ageing effect, said composition being intended to improve the appearance and quality of the skin and to improve its hydration by regulation of the water fluxes and the water absorption in the epidermis, **characterized in that** it contains a cosmetically effective amount, for the intended purpose, of an association comprising one part by weight of at least one ecdysteroid and 2 to 4 parts by weight, preferably 3 parts by weight, of at least one iridoid.

23. Composition according to claim 22, **characterized in that** said ecdysteroid is selected from the group consisting of ecdysterone, ecdysterone 2,3-monoacetonide, turkesterone, cyasterone, 22-acetylcyasterone and alpha-ecdysone.

24. Composition according to claim 22 or 23, **characterized in that** said iridoid is selected from the group consisting of harpagide and its 8-O-acetyl derivative.

25. Composition according to one of claims 22 to 24, **characterized in that** the ecdysteroids present in said association are composed of 30 to 35% of ecdysterone, 20 to 25% of turkesterone, 20 to 24% of 22-acetylcyasterone, 11 to 15% of ecdysterone 2,3-monoacetonide, 6 to 7% of alpha-ecdysone and 2 to 4% of cyasterone, the percentages being expressed by weight.

26. Composition according to one of claims 22 to 25, **characterized in that** the iridoids present in said association are composed of 60 to 70% of 8-O-acetyl-harpagide and 30 to 40% of harpagide, the percentages being expressed by weight.

27. Composition according to one of claims 21 to 26, **characterized in that** it contains from 0.005 to 10% by weight, preferably from 0.01 to 2% by weight, based on the total weight of said composition, of an extract as defined in one of claims 1 to 20 or an association as defined in one of claims 22 to 26.

28. Composition according to one of claims 21 to 27, **characterized in that** it also contains at least one substance selected from the group comprising vitamin A and its esters, particularly vitamin A palmitate and acetate, vitamin E and its esters, particularly vitamin E phosphate and acetate, vitamin C and its derivatives, particularly magnesium ascorbylphosphate, xanthines, particularly caffeine, asiatic acid, madecassic acid, asiaticosides, madecassosides, extracts of Centella asiatica, ellagic acid, soya saponins, extracts of Bertholletia, extracts of Panax Ginseng, pure sea water, magnesium aspartate, manganese chloride, cyclic AMP, D-xylose, hyaluronic acid, calcium gluconate, isoflavones, ammonium glycyrrhizinate, corticosteroids, extracts of Phellodendron amurense, resveratrol and piceids.

29. Non-therapeutic use of an extract according to one of claims 1 to 20 or an association as defined in one of claims 22 to 26 as a cosmetic agent for improving the differentiation of keratinocytes.

30. Non-therapeutic use of an extract according to one of claims 1 to 20 or an association as defined in one of claims 22 to 26 as a cosmetic agent for regulating the water fluxes and the water absorption in the epidermis.

31. Non-therapeutic use of an extract according to one of claims 1 to 20 or an association as defined in one of claims 22 to 26 as a cosmetic agent for hydrating the epidermis.

32. Method of cosmetic skin treatment, **characterized in that** a cosmetically effective amount of an extract according to one of claims 1 to 20 or an association as defined in one of claims 22 to 26 is applied to the skin.
